(19) Europäisches Patentamt
European Patent Office
Office européen des brevets

(11) Publication number: **0 154 117**
**B1**

(12) # EUROPEAN PATENT SPECIFICATION

(45) Date of publication of patent specification: **27.12.89**

(51) Int. Cl.⁴: **A 61 K 31/135**, A 61 K 31/42, A 61 K 31/395, C 07 C 97/26

(21) Application number: **85100307.9**

(22) Date of filing: **14.01.85**

(54) **Use of 1,4 bis(substituted) anthrachinones for the manufacture of immunosuppresiva.**

(30) Priority: **27.02.84 US 583540**

(43) Date of publication of application:
**11.09.85 Bulletin 85/37**

(45) Publication of the grant of the patent:
**27.12.89 Bulletin 89/52**

(84) Designated Contracting States:
**AT BE CH DE FR GB IT LI NL SE**

(56) References cited:
**DE-A-2 835 661**
**GB-A-2 000 029**
**US-A-4 278 605**
**US-A-4 410 524**
**US-A-4 428 882**

**B Helwig. Moderne Arzneimittel, pp 76, 1576(1980)**

**The file contains technical information submitted after the application was filed and not included in this specification**

(73) Proprietor: **AMERICAN CYANAMID COMPANY**
**1937 West Main Street P.O. Box 60**
**Stamford Connecticut 06904-0060 (US)**

(72) Inventor: **Murdock, Keith Chadwick**
**15 Birch Street**
**Pearl River New York 10965 (US)**
Inventor: **Durr, Frederick Emil**
**387 Jefferson Street**
**Ridgewood New Jersey 07450 (US)**
Inventor: **Wang, Bosco Shang**
**59 Verdin Drive**
**New City New York 10956 (US)**

(74) Representative: **Wächtershäuser, Günter, Dr.**
**Tal 29**
**D-8000 München 2 (DE)**

(56) References cited:
**B Helwig. Moderne Arzneimittel, pp 76, 1576(1980)**

**Journal of Medic. Chemistry, vol 22 no. 7 July 1979. Murdoch et al "Antitumor agents. 1.1,4-bis (aminoalkyl amino)-9, 10-anthracenediones" pp. 1024-1030**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 154 117 B1

## Description

This invention is concerned with a method of inducing immunosuppression in warm-blooded animals by the parenteral administration of a compound selected from those of the formula:

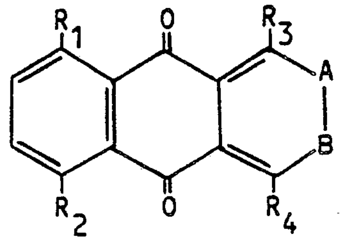

wherein $R_1$ and $R_2$ and are both hydroxy; A—B is —CH=CH—; $R_3$ and $R_4$ may be the same or different and are:

$$—NHCH_2CH_2NH_2 \text{ or } —NHCH_2CH_2NHCH_2CH_2OH;$$

either as the free base or as the pharmacologically acceptable acid addition salts thereof.

1,4-Bis[([2-aminoethyl)amino]-5,8-dihydroxyanthraquinone, dihydrochloride; and 1,4-dihydroxy-5,8-bis[[2-(2-hydroxyethylamino)ethyl]amino]anthraquinone, dihydrochloride are disclosed in U.S. Patents i.e. US—A—4,197,249 or 4,278,689; or by Wallace, R. E., *et al.,* Current Chemotherapy and Immunotherapy, 1363 (1981); or by Murdock, K. C., *et al.,* J. Med. Chem., *22,* 1024 (1979).

In the above listed patents and publications these compounds are described as anti-tumor agents.

It has now been discovered that the above described compounds are extremely potent as immunosuppressive agents when administered parenterally to warm-blooded animals.

As such they are effective in treating conditions where elevated levels of antibody production or monocytelymphocyte activity as a result of the hyperreactivity of the immunoregulatory network are closely associated with the development of autoimmune diseases, including rheumatoid arthritis [Mellbye, O. J. and Natvig, J. B. Clin. Exp. Immunol., *8,* 899 (1971), multiple sclerosis [Tourtellote, W. W. and Parker, J. A., Science, 154, 1044 (1966)], systemic lupus erythematosis [Abdou, N. I., *et al.,* Clin. Immonol. Immunopath., *6,* 192 (1976)], thyroiditis [Witebsky, E., *et al.,* J. Immunol., *103,* 708 (1969)], mixed connective tissue disease [Sharp, G. C., *et al.,* Am. J. Med., *52,* 148 (1972)], dermato/polymyositis [Venables, P. J. W., *et al.,* Ann. Rheum. Dis., *40,* 217 (1981), insulin-dependent diabetes [Charles, M. A., *et al.,* J. Immunol., 130, 1189 (1983)] and in patients undergoing organ transplantation.

Subject of the present invention is the use of the above-described compounds either as the free base or as the pharmacologically acceptable acid addition salt thereof for the manufacture of a medicament ready for the treatment of multiple sclerosis and rheumatoid arthritis.

Their activity was established both *in vitro* and *in vivo* in a variety of test procedures described below.

### Animals, Tumors and Reagents

C57BL/6(B6,H—$2^b$) and DBA/2(D2,H—$2^d$) mice, ages 6—10 weeks from Charles River Breeding Lab., Inc., Wilmington, MA, and Jackson Lab., Bar Harbor, ME, were used.

P815 Mastocytoma cells (H—$2^d$) served as target cells for cytotoxicity assays. They were maintained by weekly intraperitoneal (i.p) passage in D2 mice in an ascites form.

Hanks' balanced salt solution (HBSS), Roswell Park Memorial Institute (RPMI) 1640 medium, fetal calf serum (FCS), L-glutamine, penicillin, streptomycin, Dulbecco's phosphate buffered saline (D—PBS) and N-2-hydroxyethylpiperazine-N'-2-ethanesulfonic acid (HEPES) were obtained from Grand Island Biological Co., Grand Island, NY. Gentamicin was obtained from Schering Corp., Kenilworth, NJ. Concanavalin A (Con A), ammonium chloride and trichloroacetic acid (TCA) were obtained from Sigma Chemical Co., St. Louis, MO. 2-Mercaptoethanol was obtained from Eastman Kodak Co., Rochester, NY. Lipopolysaccharide (LPS, *E. coli* 0128:B12) was obtained from DIFCO Lab., Detroit, MN. Both methyl-$^3$H-thymidine ($^3$H-TdR, specific activity = 20 Ci/mmol) and $^{51}$Cr (specific activity = 200—500 Ci/g chromium) were obtained from New England Nuclear, Boston, MA.

### Preparation of Mouse Lymphocytes

Spleens were removed from mice and single cell suspensions were prepared by teasing the spleens apart and rinsing through No. 40 and No. 80 stainless steel mesh screens. Erythrocytes were lysed by a 3 minute exposure to a 0.83% Tris-ammonium chloride solution. Cells were washed three times with HBSS and finally suspended in RPMI 1640 culture medium containing 5% FCS, 2mM L-glutamine, $5\times10^{-5}$M 2-mercaptoethanol, 10mM HEPES, 100 μ/ml of penicillin, 100 μg/ml of streptomycin and 50 μ/ml of gentamicin (referred to as complete medium). Cells were counted in a hemocytometer and the viability was always greater than 90% as judged by trypan blue dye exclusion.

Tests

Proliferative Responses of Lymphocytes to Con A and LPS

$2\times10^5$ Mouse lymphocytes were dispensed in flat-bottom wells of a 96-well culture plate (Coster, Cambridge, MA), Con A and LPS were also added to these cultures at predetermined optimal concentrations of 1 µg/ml and 10 µg/ml, respectively. The medium volume was 0.2 ml per culture. After being incubated at 37°C, in a 7% carbon dioxide, water saturated atmosphere for 3 days, these cells were pulsed with 0.5 µg/ml of $^3$H-TdR for the final 4 hours of incubation and harvested by a cell harvester. Cellular DNA was precipitated on glass fiber filters with 10% TCa and the amount of $^3$H-TdR incorporated was determined in a Beckman liquid scintillation counter. Cultures were always performed in triplicate.

Proliferative Response of Mouse Lymphocytes to Alloantigens in Mixed Lymphocyte Cultures (MLC)

$2\times10^5$ B6 Responding lymphocytes were cultured together with $2\times10^5$ D2 stimulating lymphocytes, which had been irradiated with 2,000 rads of gamma radiation, in 0.2 ml of complete medium in the flat-bottom wells of a 96-well Costar culture plate. The plates were incubated at 37°C, in a 7% carbon dioxide, water saturated atmosphere for 5 days. Cells were pulsed with $^3$H—TdR for the final 4 hours of incubation and the amount of $^3$H-TdR incorporated was similarly determined by the hereinabove described method.

Induction of Alloreactive Cytolytic T Lymphocytes (CTL) in MLC

$10^7$ C57BL/6 Mouse lymphocytes were co-cultured with $8\times10^6$ irradiated DBA/2 mouse lympocytes in 4 ml of complete medium in $17\times100$ mm Falcon culture tubes. The test compounds were initially dissolved in dimethylsulfoxide (DMSO) and diluted to proper concentrations with culture medium immediately prior to testing. All compounds were tested at concentrations of $10^{-3}$ to $10^{-6}$ µg/ml at which the compounds themselves caused no direct toxicity to lymphocytes. The final content of DMSO was $\leq2\times10^{-5}$ of the original and was shown to have no effect in this system. The MLC were incubated at 37°C for 5 days and sensitized cytollytic T lymphocytes (CTL) were harvested and examined for their cytotoxicity in $^{51}$Cr-release assay using P815 mastocytoma cells as targets. By comparison with the cytotoxicity of CTL from control MLC, containing no compound, % suppression of the generation of CTL by the test compound was calculated by the formula:

$$\% \text{ Suppression} = \left(1 - \frac{A}{B}\right. \times 100,$$

where A = CTL activity from MLC containing test compound and B = CTL activity from control MLC.

The results of this test on representative compounds of this invention appear in Table III.

Induction of Alloreactive CTL in Animals

B6 Mice were sensitized by i.p. injection of $2\times10^7$ viable allogeneic P815 cells and subsequently challenged with the same number of P815 cells 10 days later. Lymphocytes were prepared from these mice 2 days after challenge and tested for cytotoxicity.

$^{51}$Cr-release Microcytotoxicity Assay

P815 Tumor cells were harvested from D2 mouse peritoneal cavities. After being washed once with HPSS, $10^7$ tumor cells were incubated with 10 µCi $^{51}$Cr in 0.5 ml of HBSS at 37°C for 60 minutes. Cells were then washed three times and suspended in RPMI 1640 medium containing 10% FCS. $10^4$ Of these $^{51}$Cr-labeled target cells in 0.05 ml were added to each V-shaped well of a Linbro microtiter plate (Linbro Chemical Co., Hamden, CT). Effector cells generated in 5-day MLC or from sensitized animals in 0.1 ml of medium were also added to each well at various effector to target cell (E:T) ratios. Unless otherwise noted, results are presented from cultures with an E:T ratio of 40:1. Microtiter plates were centrifuged at $40\times G$ for 3 minutes and then incubated at 37°C for 3.5 hours. After incubation, the plates were centrifuged at $600\times G$ for 5 minutes, then 0.1 ml of culture medium was carefully removed and the released radioactivity was determined in a Packard gamma counter. The cytotoxicity (as percent lysis) was calculated from triplicate cultures by the formula

$$\% \text{ lysis} = \frac{(E-S)(100)}{(M-S)},$$

where E = cpm of $^{51}$Cr spontaneously released from labeled target cells; and M = maximum cpm of $^{51}$Cr released from labeled target cells.

The results were analyzed statistically. The significance of the difference between control and experimental samples was determined by Student's *t* test.

Results

Inhibition of the Proliferative Response of Lymphocytes to Mitogens and Alloantigens

$2\times10^5$ B6 Lymphocytes were stimulated with Con A or LPS *in vitro*. 1,4-Bis[(2-aminoethyl)amino]-5,8-dihydroxyanthraquinone dihydrochloride was added at various concentrations to the cultures and its inhibitory effect was determined 3 days later by the reduced amounts of $^3$H-TdR incorporated into cellular DNA. The results appear in Table I, and show that the blast transformation of mouse lymphocytes in response to Con A and LPS significantly decreased with $10^{-3}$ and $10^{-4}$ μg/ml of 1,4-bis[(2-aminoethyl)amino]-5,8-dihydroxyanthraquinone dihydrochloride, respectively. Furthermore, the proliferation of these cells was completely abolished by this compound at 1 to $10^{-2}$ μg/ml without altering their viability as determined by trypan blue dye exclusion.

Alloreactivity of lymphocytes was studied in a one-way, 5-day MLC. $B_6$ Lymphocytes were stimulated by D2 lymphocytes and underwent active proliferation as shown in Table II. However, addition of $10^{-2}$ and $10^{-4}$ μg/ml of 1,4-bis(2-aminoethyl)amino]-5,8-dihydroxyanthraquinone dihydrochloride significantly prevented this reaction from taking place.

Inhibition of CTL Induction *in vitro*

CTL were generated in MLC by mixing B6 with irradiated D2 lymphocytes and 1,4-bis[(2-aminoethyl)amino]-5,8-dihydroxyanthraquinone dihydrochloride was added to some cultures at various concentrations. Effector CTL were harvested from these MLC 5 days later and tested for cytotoxicity using $^{51}$Cr-labeled P815 cells as targets. A concentration of $10^{-5}$ mcg/ml of this compound significantly decreased CTL activity (% lysis = 37.0; $p < 0.01$) when compared to control CTL (% lysis = 45.7). A complete failure to generate CTL was observed with 1 to $10^{-4}$ μg/ml of the compound.

In order to determine the time necessary to induce immunosuppression, $10^{-3}$ μg/ml of 1,4-bis[(2-aminoethyl)amino]-5,8-dihydroxyanthraquinone dihydrochloride was added to MLC at different time intervals. As shown in Table IV, CTL was almost totally absent in cultures when this compound was added at the same time as (% lysis = 2.7) or one day after (% lysis = 6.4) the initiation of the experiment. CTL became detectable when the compound was added 2 days after setting up MLC (% lysis = 22.0), but the activity was still significantly less than that of control (% lysis = 52.0; $p < 0.001$).

The duration of the suppressive effect of 1,4-bis[(2-aminoethyl)amino]-5,8-dihydroxyanthraquinone dihydrochloride was studied by adding this compound to cultures at $10^{-3}$ μg/ml and then removing the compound from 1 MLC each day by thoroughly washing the cells with HBSS and reculturing in fresh medium. Five days after initiation of the test, CTL were harvested and tested for cytotoxicity. The results, shown in Table V, clearly indicate that the suppression induced by this compound was irreversible, regardless of when the compound was removed from the cultures.

Immunosuppression by 1,4-bis[(2-aminoethyl)amino]-5,8-dihydroxyanthraquinone dihydrochloride was compared on a weight to weight basis with adriamycin, cyclosporin A and Novantrone ™ [1,4-dihydroxy-5,8-bis[[2-(2-hydroxyethylamino)ethyl]anthraquinone dihydrochloride (U.S. Patent 4,197,249)]. As shown in Table VI, when compared to control CTL generated in drug-free MLC, the minimum doses required to cause a significant effect were:

| | |
|---|---|
| Novantrone™ | $10^{-3}$ μg/ml |
| Adriamycin | $10^{-1}$ μg/ml |
| Cyclosporin A | $10^{-2}$ μg/ml |

A consistent failure to generate CTL by 1,4-bis[(2-aminoethyl)amino]-5,8-dihydroxyanthraquinone dihydrochloride at $10^{-4}$ mcg/ml shows the superior potency of this compound in depressing alloreactivity.

The evidence present in Tables I—VI show that 1,4-bis[(2-aminoethyl)amino]-5,8-dihydroxyanthraquinone dihydrochloride is highly effective in preventing the induction of CTL *in vitro*.

Immunosuppression *in vivo*

B6 Mice were treated i.p. with 3 mg/kg of body weight of 1,4-bis[(2-aminoethyl)amino]-5,8-dihydroxyanthraquinone dihydrochloride and lymphocytes were prepared from these mice 2 weeks later. These cells were stimulated in MLC with D2 stimulatory cells for 5 days and the CTL were harvested and tested for cytotoxicity. As shown in Table VII as compared to CTL generated in normal untreated mice, lymphocytes from drug-treated mice failed to become effective CTL at all E:T ratios tested.

The optimal *in vivo* dose and timing of the treatment was determined by treating B6 mice with 0.3 to 3.0 mg/kg of body weight of the test drug and MLC were set up from these mice at various time intervals. The results in Table VIII show that at day 10 after treatment lymphocytes from all treated mice failed to respond to alloantigens *in vitro*, thus showing no CTL activity. At day 23 post treatment, a typical CTL generation was evident only in normal mice and mice treated with 0.3 mg/kg of the test compound. After 30 days of treatment, effector cells generated in mice receiving 0.3 and 0.75 mg/kg test compound manifested a normal level of cytotoxicity whereas lymphocytes from mice treated with 1.5 an 3.0 mg/kg of test compound were still unable to do so, proving the potent and long lasting inhibitory effect of this compound.

Evidence for the Presence of Suppressor Cells in Treated Mice

B6 Mice were treated with 3 mg/kg of body weight with 1,4-bis[(2-aminoethyl)amino]-5,8-dihydroxyanthraquinone dihydrochloride and lymphocytes were obtained 5 weeks later. These cells were added to a conventional MLC consisting of $10^7$ normal B6 lymphocytes and $5\times10^6$ irradiated D2 lymphocytes. As shown in Table IX, addition of 1—$5\times10^6$ cells from drug treated mice significantly prevented normal lymphocytes from becoming effective CTL indicating the existence of suppressor cells in the spleens of treated mice, and effect which is dose related.

To summarize, the compounds of this invention have a potent suppressive effect on cellular immune responses *in vitro* and *in vivo*. Addition of one such compound to cultures significantly decreased the proliferation responses of lymphocytes to Con A, LPS and alloantigens (Tables I and II). A complete inhibition of proliferation demonstrated with 1 to $10^{-2}$ µg/ml of this compound was not the result of direct toxicity since the viability of treated cells remained unaltered as judged by trypan blue dye exclusion. Induction of alloreactive CTL was also significantly lessened by all of these compounds in MLC (Table III). In order to achieve a significant effect the compound had to be added within the first 3 days of a 5 day MLC indicating that the inductive phase was the period most susceptible (Table IV). The irreversible effect was also evidenced by the failure of pretreated lymphocytes to respond to antigens after the compound was removed (Table V). This contributes to an unusual long lasting immunoincompetence in animals (Table VIII). These compounds are clearly superior to Novantrone™, adriamycin and cyclosporin A in depressing CTL induction (Table VI). This is particularly significant in view of the fact that cyclosporin A has been proven clinically useful in organ transplantation (Calme, R.Y., Immunol. Rev., *46*, 113 (1979)].

TABLE I

Inhibitary Effect of 1,4-Bis[(2-aminoethyl)amino]-5,8-dihydroxyanthraquinone Dihydrochloride on the Proliferative Responses of Lymphocytes to Con A and LPS

| Concentration µg/ml | $^3$H-TdR Incorporation (cpm ± SD)* | |
|---|---|---|
| | Con A Stimulation | LPS Stimulation |
| 0 (Control) | 66404.0 ± 7047.7 | 25221.3 ± 2377.3 |
| 1 | 392.0 ± 25.5** | 326.5 ± 149.2** |
| $10^{-}$ | 405.0 ± 48.1** | 296.0 ± 125.9** |
| $10^{-2}$ | 245.5 ± 185.9** | 300.3 ± 132.1** |
| $10^{-3}$ | 18200.3 ± 1377.8** | 3492.5 ± 400.9** |
| $10^{-4}$ | 63427.5 ± 4910.9 | 11998.7 ± 2136.8** |
| $10^{-5}$ | 67106.0 ± 11160.0 | 21330.5 ± 2370.9 |

*The test compound was added at various concentrations to $2\times10^5$ B6 lymphocytes stimulated with 1 µg/ml of Con A or 10 µg/ml of LPS. Cpm of unstimulated lymphocytes = 1304.3 ± 109.5.
**Statistically significant when compared to control cpm ($p < 0.001$).

TABLE II

Inhibitary Effect of 1,4-Bis[(2-aminoethyl)amino]-
5,8-dihydroxyanthraquinone Dihydrochloride
on the Proliferative Responses of Lymphocytes to Alloantigens

| Concentration μg/ml* | $^3$H-TdR Incorporation (cpm ± SD) | P |
|---|---|---|
| C (Control) | 5651.0 ± 835.1 | |
| $10^{-2}$ | 524.5 ± 105.4** | <0.001 |
| $10^{-4}$ | 3640.5 ± 19.1** | <0.01 |
| $10^{-6}$ | 6607.0 ± 387.5 | |
| $10^{-8}$ | 5823.0 ± 120.2 | |

*The test compound was added at various concentrations to 5-day MLC consisting of $2\times10^5$ B6 lymphocytes and $2\times10^5$ irradiated D2 lymphocytes. Cpm of B6 lymphocytes alone = 871.7 ± 135.2.
**Statistically significant.

TABLE III

Inhibition of the Induction of CTL *In Vitro*

| Compound | Concentrations Which Induced ≥50% Suppression |
|---|---|
| 1,4-Bis[(2-aminoethyl)amino]-5,8-dihydroxy-anthraquinone, dihydrochloride | $10^{-6}$ |
| 1,4-Dihydroxy-5,8-bis[[2-(2-hydroxyethylamino)-ethyl]amino]anthraquinone, dihydrochloride | $10^{-4}$, $10^{-5}$ |

TABLE IV

Time Course of the Inhibition of CTL Induction *In Vitro* With
1,4-Bis[(2-aminoethyl)amino]-5,8-dihydroxyanthraquinone, Dihydrochloride

| Time of Addition (Day post MLC initiation)* | % Lysis ± SEM** | P |
|---|---|---|
| Control*** | 52.0 ± 2.6 | |
| 0 | 2.7 ± 0.8 | <0.001 |
| 1 | 6.4 ± 1.0 | <0.001 |
| 2 | 22.0 ± 0.9 | <0.001 |
| 3 | 60.8 ± 1.8 | <0.01 |
| 4 | 70.6 ± 1.4 | <0.01 |

*The test compound was added to MLC at a final concentration of $10^{-3}$ μg/ml.
** Lysis was determined by $^{51}$Cr-release assay using P815 cells as targets.
***Control MLC contained no test compound.

TABLE V

Persistent Effect of 1,4-Bis[(2-aminoethyl)amino]-5,8-dihydroxy-anthraquinone Dihydrochloride on Inhibition of CTL Induction *In Vitro*

| Time of Drug Removal* (day post MLC initiation) | % Lysis ± SEM** | P |
|---|---|---|
| Control | 61.3 ± 6.5 | |
| 1 | 10.1 ± 1.5 | ± 0.01 |
| 2 | 0.2 ± 0.2 | ± 0.001 |
| 3 | 0 | ± 0.001 |
| 4 | 0 | ± 0.001 |

*The test compound ($10^{-3}$ µg/ml) was initially added to all MLC except the control. The test compound was removed from one MLC each day as indicated and recultured in fresh medium.
**Lysis was determined by $^{51}Cr$-release assay using P815 cells as targets.

TABLE VI

Inhibition of CTL Induction *In Vitro* by 1,4-Bis[(2-aminoethyl)amino]-5,8-dihydroxyanthraquinone Dihydrochloride.
Comparison With the Effects of Novantrone™, Adriamycin and Cyclosporin A*

| Concentration (µg/ml) | Test 1 | | Test 2 | | Test 3 | |
|---|---|---|---|---|---|---|
| | Test Drug | Novantrone™ | Test Drug | Adriamycin | Test Drug | Cyclosporin A |
| 1 | NT | 1.9 ± 0.6** | NT | 0** | 1.6 ± 0.7** | 0** |
| $10^{-1}$ | NT | 0.5 ± 0.6** | NT | 7.9 ± 0.6** | 0 | 0.1 ± 0.1** |
| $10^{-2}$ | 1.7 ± 0.2** | 0.9 ± 0.6** | 0** | 21.9 ± 3.8 | 0 | 63.9 ± 2.0**** |
| $10^{-3}$ | 0.6 ± 0.4** | 23.0 ± 0.9** | 0** | NT | 0.1 ± 0.1** | 67.5 ± 0.5 |
| $10^{-4}$ | 12.9 ± 1.6** | 47.1 ± 0.7 | 14.1 ± 0.7*** | 21.9 ± 0.9 | 0 | 70.1 ± 1.2 |
| Control***** | 46.8 ± 0.9 | | 20.8 ± 0.8 | | 71.5 ± 1.1 | |

*The test drugs, Novantrone™, Adriamycin and Cyclosporin A were added to MLC and effector CTL were harvested from these MLC 5 days later and tested for cytotoxicity in $^{51}Cr$-release assays.
NT = not tested.
**$P < 0.001$ compared to control.
***$P < 0.01$ compared to control.
****$P < 0.05$ compared to control.
*****Control MLC contained no test drug.

TABLE VII

Immunosuppressive Activity of 1,4-Bis[(2-aminoethyl)amino]-5,8-dihydroxyanthraquinone Dihydrochloride *In Vivo*

| E:T Ratios | % Lysis ± SEM* | | p*** |
|---|---|---|---|
| | Normal | Drug Treated** | |
| 20:1 | 39.2 ± 1.0 | 4.5 ± 4.4 | < 0.01 |
| 10:1 | 28.9 ± 0.9 | 0 | < 0.001 |
| 5:1 | 17.3 ± 0.9 | 0.2 ± 0.2 | < 0.001 |

*Lysis was determined by $^{51}Cr$-release assay using P815 cells as targets.
**B6 mice were treated i.p. with 3 mg/kg of test drug 2 weeks before testing.
***P values were determined by comparison of CTL activities between normal and drug treated mouse lymphocytes.

TABLE VIII

Immunosuppressive Activity of 1,4-Bis[(2-aminoethyl)amino]-5,8-dihydroxyanthraquinone Dihydrochloride *In Vivo*

| Dos* (mg/kg) | % Lysis ± SEM | | |
|---|---|---|---|
| | 10 days Post Treatment | 23 Days Post Treatment | 30 Days Post Treatment |
| 0 (Control) | 51.4 ± 1.2 | 74.7 ± 2.1 | 66.5 ± 0.4 |
| 0.3 | 4.1 ± 0.2** | 70.3 ± 1.2 | 66.4 ± 3.2 |
| 0.75 | 0.5 ± 0.5** | 1.3 ± 0.8** | 65.7 ± 1.0 |
| 1.5 | 1.3 ± 0.4** | 0.1 ± 0.1** | 2.0 ± 0.7** |
| 3.0 | 0.7 ± 0.2** | 0** | 2.4 ± 0.3** |

*B6 mice were treated i.p. with various doses of the test compound. Lymphocytes were prepared from these mice 10, 23 and 30 days after treatment and cultured in MLC in order to generate CTL.
**P < 0.001 compared to control.

TABLE IX

Evidence for the Presence of Suppressor Cells in Mice Treated with 1,4-Bis[(2-aminoethyl)amino]-5,8-dihydroxyanthraquinone Dihydrochloride

| Addition of Drug-treated Mouse Lymphocytes to Normal MLC* | % Lysis ± SEM | P |
|---|---|---|
| None (Control) | 61.3 ± 0.9 | |
| $5 \times 10^5$ | 57.9 ± 1.6 | |
| $1 \times 10^6$ | 51.5 ± 1.8 | < 0.05 |
| $3 \times 10^6$ | 39.9 ± 0.7 | |
| 5 × 10.309 | 15.1 ± 0.7 | < 0.001 |

*B6 mice were injected with 3 mg/kg of test compound. Lymphocytes were obtained from these treated mice 5 weeks later and added to MLC consisting of $10^7$ normal B6 lymphocytes and $5 \times 10^6$ irradiated D2 lymphocytes.

This aspect of the invention includes novel compositions of matter containing the compounds of this invention and the method of inducing suppression of the immune response system in mammals when administered in amounts ranging from about one mg to about 50 mg per square meter of body surface area per day. The interrelationship of dosages for animals of various sizes and species and humans (based on $mg/m^2$ of surface area) is described by Freireich, E. J., *et al.,* Quantitative Comparison of Toxicity of Anticancer Agents in Mouse, Rat, Hamster, Dog, Monkey and Man. Cancer Chemother. Rep., *50,* No. 4, 219—244, May 1966. A preferred dosage regimen for optimum results would be from 3 $mg/m^2$/day to 50 $mg/m^2$/day, and such dosage units are employed that a total of from 5 mg to 125 mg of the active compound for a subject of 70 kg of body weight are administered in a 24 hour period. This dosage regimen may be adjusted to provide the optimum therapeutic response. For example, several divided doses may be administered daily or the dose may be proportionally reduced as indicated by the exigencies of the therapeutic situation. The active compound may be administered by the intravenous, intramuscular, or subcutaneous routes.

The active compounds may be administered parenterally. Solutions or dispersions of the active compound can be prepared in water suitably mixed with a surfactant such as hydroxypropylcellulose. Dispersions can also be prepared in glycerol, liquid polyethylene glycols, and mixtures thereof and in oils. Under ordinary conditions of storage and use, these preparations contain a preservative to prevent the growth of microorganisms.

The pharmaceutical forms suitable for injectable use include sterile aqueous solutions or dispersions and sterile powders for the extemporaneous preparation of sterile injectable solutions or dispersions. In all cases the form must be sterile and must be fluid to the extent that easy syringability exists. It must be stable under the conditions of manufacture and storage and must be preserved against the contaminating action of microorganisms such as bacteria and fungi. The carrier can be a solvent or dispersion medium containing, for example, water, ethanol, polyol (for example, glycerol, propylene glycol, and liquid polyethylene glycol), suitable mixtures thereof, and vegetable oils. The proper fluidity can be maintained, for example, by the use of a coating such as lecithin, by the maintenance of the required particle size in the case of dispersion and by the use of surfactants. The prevention of the action of microorganisms can be brought about by various antibacterial and antifungal agents, for example, parabens, chlorobutanol, phenol, sorbic acid, thimerosal, and the like. In many cases it will be preferable to include isotonic agents, for example, sugars or sodium chloride. Prolonged absorption of the injectable compositions can be brought about by the use in the compositions of agents delaying absorption, for example, aluminum monosterate and gelatin. Sterile injectable solutions are prepared by incorporating the active compound in the required amount in the appropriate solvent with various of the other ingredients enumerated above, as required, followed by filtered sterilization. Generally, dispersions are prepared by incorporating the various sterilized active ingredient into a sterile vehicle which contains the basic dispersion medium and the required other ingredients from those enumerated above. In the case of sterile powders for the preparation of sterile injectable solutions, the preferred methods of preparation are vacuum drying and the freeze-drying technique which yield a powder of the active ingredient plus any additional desired ingredient from a previously sterile-filtered solution thereof.

As used herein, "pharmaceutically acceptable carrier" includes any and all solvents, dispersion media, coatings, antibacterial and antifungal agents, isotonic and absorption delaying agents. The use of such media and agents for pharmaceutically active substances is well known in the art. Except insofar as any conventional media or agent is incompatable with the active ingredient, its use in the therapeutic compositions is contemplated. Supplementary active ingredients can also be incorporated into the compositions.

It is especially advantageous to formulate parenteral compositions in dosage unit form for ease of administration and uniformity of dosage. Dosage unit form as used herein refers to physically discrete units suited as unitary dosages for the mammalian subjects to be treated; each unit containing a predetermined quantity of active material calculated to produce the desired therapeutic effect in association with the required pharmaceutical carrier. The specification for the novel dosage unit forms of the invention are dictated by and directly dependent on (a) the unique characteristics of the active material and the particular therapeutic effect to be achieved, and (b) the limitations inherent in the art of compounding such an active material for the treatment of disease in living subjects having a diseased condition in which bodily health is impaired as herein disclosed in detail.

The principal active ingredient is compounded for convenient and effective administration in effective amounts with a suitable pharmaceutically acceptable carrier in dosage unit form as hereinbefore disclosed. A unit dosage form can, for example, contain the principal active compound in amounts ranging from 2 mg to 200 mg, with from 5 to 125 mg being preferred. Expressed in proportions, the active compound is generally present in from 2 to 100 mg/ml of carrier. In the case of compositions containing supplementary active ingredients, the dosages are determined by reference to the usual dose and manner of administration of the said ingredients.

Suppression of the immune system is attained, for example, using parenteral administration. A single intravenous dosage or repeated daily dosages can be administered. Daily dosages up to 5 or 10 days are often sufficient. It is also possible to dispense one daily dosage or one dose on alternate or less frequent days. As can be seen from the dosage regimens, the amount of principal active ingredient administered is a

sufficient amount to induce and enhance immunosuppression, in the absence of excessive deleterious side effects of a cytotoxic nature to the hosts.

**Claim**

Use of a compound selected from those of the formula:

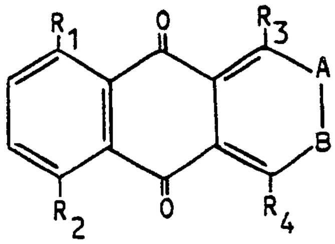

wherein $R_1$ and $R_2$ are both hydroxy; A—B is —CH=CH—; and $R_3$ and $R_4$ are the same and are:

$$—NHCH_2CH_2NH_2 \text{ or } —NHCH_2CH_2NHCH_2CH_2OH;$$

either as the free base or as the pharmacologically acceptable acid addition salts thereof for the manufacture of a medicament ready for the treatment of multiple sclerosis and rheumatoid arthritis.

**Patentanspruch**

Verwendung einer Verbindung, ausgewählt aus solchen der Formel

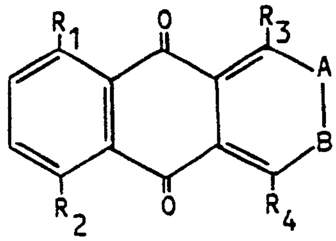

wobei $R_1$ und $R_2$ beide Hydroxy sind; A—B für —CH=CH— steht; und $R_3$ und $R_4$ gleich sind und für

$$—NHCH_2CH_2NH_2 \text{ oder } —NHCH_2CH_2NHCH_2CH_2OH;$$

stehen,
entweder als die freie Base oder als das pharmazeutische Säureadditionssalz derselben bei der Herstellung eines Medikaments für die Behandlung von multipler Sklerose und rheumatoider Arthritis.

**Revendication**

Utilization d'un composé choisi parmi ceux de formule:

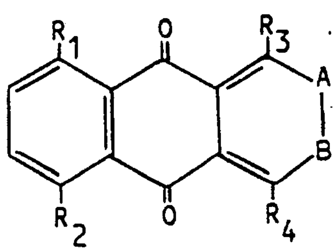

dans laquelle $R_1$ et $R_2$ sont sout les deux des groupes hydroxy; A—B est —CH=CH—; et $R_3$ et $R_4$ sont identiques et désignent:

$$—NHCH_2CH_2NH_2 \text{ ou } —NHCH_2CH_2NHCH_2CH_2OH;$$

ce composé étant sous la forme de base libre ou sous la forme de sels d'addition d'acides pharmacologiquement acceptable pour la fabrication d'un médicament prêt pour le traitement de la sclérose multiple et du rhumatisme articulaire.